(19) Europäisches Patentamt
European Patent Office
Office européen des brevets

(11) **EP 3 988 169 B1**

(12) **EUROPEAN PATENT SPECIFICATION**

(45) Date of publication and mention of the grant of the patent:
**29.04.2026 Bulletin 2026/18**

(21) Application number: **21203094.4**

(22) Date of filing: **18.10.2021**

(51) International Patent Classification (IPC):
***A61P 1/00*** *(2006.01)* ***A23C 21/00*** *(2025.01)*
***A23J 1/20*** *(2006.01)*

(52) Cooperative Patent Classification (CPC):
(C-Sets available)
**A61P 1/00; A23C 21/00; A23J 1/205;**
A23V 2002/00 (Cont.)

# (54) NUTRACEUTICAL COMPOSITION COMPRISING BUFFALO MILK WHEY, AND USE THEREOF IN THE REDUCTION OF THE INTESTINAL PERMEABILITY

NUTRACEUTISCHE ZUSAMMENSETZUNG, DIE BÜFFELMILCHMOLKE UMFASST, UND DEREN VERWENDUNG BEI DER REDUZIERUNG DER INTESTINALPERMEABILITÄT

COMPOSITION NUTRACEUTIQUE COMPRENANT DU LACTOSÉRUM DE LAIT DE BUFFLONNE ET SON UTILISATION DANS LA RÉDUCTION DE LA PERMÉABILITÉ INTESTINALE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.10.2020 IT 202000024742**

(43) Date of publication of application:
**27.04.2022 Bulletin 2022/17**

(73) Proprietors:
- **Consorzio Per La Tutela Del Formaggio Mozzarella Di Bufala Campana**
  **81100 Caserta (CE) (IT)**
- **Tenore, Gian Carlo**
  **80128 Napoli (IT)**
- **Novellino, Ettore**
  **83100 Avellino (IT)**

(72) Inventors:
- **TENORE, Gian Carlo**
  **80128 Napoli (IT)**
- **NOVELLINO, Ettore**
  **83100 Avellino (IT)**

(74) Representative: **Bird & Bird Società tra Avvocati S.r.l.**
**Via Porlezza, 12**
**20123 Milano (IT)**

(56) References cited:
- **DATABASE GNPD [online] MINTEL; 21 September 2020 (2020-09-21), ANONYMOUS: "Buffalo Ricotta Cream", XP055820547, retrieved from https://www.gnpd.com/sinatra/recordpage/8121171/ Database accession no. 8121171**
- **BARILE VITTORIA LUCIA ET AL: "Production of homogenized fresh Ricotta cheese from buffalo milk", 1 April 2015 (2015-04-01), XP055820569, Retrieved from the Internet <URL:file:///C:/Users/cd51463/Downloads/ProductionofhomogenizedfreshRicottacheesefrombuffalomilk_Istanbul2015%20(1).pdf> [retrieved on 20210702]**
- **DE SIMONE CARMELA ET AL: "Characterisation and cytomodulatory properties of peptides from Mozzarella di Bufala Campana cheese whey : Bioactive peptides in mozzarella di Bufala whey", vol. 15, no. 3, 26 November 2008 (2008-11-26), pages 251 - 258, XP055821798, ISSN: 1075-2617, Retrieved from the Internet <URL:https://api.wiley.com/onlinelibrary/tdm/v1/articles/10.1002%2Fpsc.1093> DOI: 10.1002/psc.1093**
- **BASILICATA MANUELA ET AL: "Antioxidant Properties of Buffalo-Milk Dairy Products: A [beta]-Lg Peptide Released after Gastrointestinal Digestion of Buffalo Ricotta Cheese Reduces Oxidative Stress in Intestinal Epithelial Cells", INTERNATIONAL JOURNAL OF MOLECULAR SCIENCES, vol. 19, no. 7, 4 July 2018 (2018-07-04), pages 1955, XP055821799, DOI: 10.3390/ijms19071955**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- DE SIMONE CARMELA ET AL: "Peptides from water buffalo cheese whey induced senescence cell death via ceramide secretion in human colon adenocarcinoma cell line", MOLECULAR NUTRITION & FOOD RESEARCH, vol. 55, no. 2, 19 August 2010 (2010-08-19), DE, pages 229 - 238, XP055821566, ISSN: 1613-4125, DOI: 10.1002/mnfr.201000074
- TENORE GIAN ET AL: "Intestinal Anti-Inflammatory Effect of a Peptide Derived from Gastrointestinal Digestion of Buffalo (Bubalus bubalis) Mozzarella Cheese", NUTRIENTS, vol. 11, no. 3, 13 March 2019 (2019-03-13), pages 610, XP055821800, DOI: 10.3390/nu11030610
- DATABASE GNPD [online] MINTEL; 31 October 2006 (2006-10-31), ANONYMOUS: "Diet Capsules", XP055821813, retrieved from https://www.gnpd.com/sinatra/recordpage/602320/ Database accession no. 602320
- BASSAN JULIANA C. ET AL: "Buffalo Cheese Whey Proteins, Identification of a 24 kDa Protein and Characterization of Their Hydrolysates: In Vitro Gastrointestinal Digestion", vol. 10, no. 10, 14 October 2015 (2015-10-14), pages e0139550, XP055821802, Retrieved from the Internet <URL:https://storage.googleapis.com/plos-corpus-prod/10.1371/journal.pone.0139550/1/pone.0139550.pdf?X-Goog-Algorithm=GOOG4-RSA-SHA256&X-Goog-Credential=wombat-sa@plos-prod.iam.gserviceaccount.com/20210706/auto/storage/goog4_request&X-Goog-Date=20210706T185945Z&X-Goog-Expires=86400&X-Goog-SignedHeaders=h> DOI: 10.1371/journal.pone.0139550
- BASILICATA MANUELA GIOVANNA ET AL: "Peptidome profiles and bioactivity elucidation of buffalo-milk dairy products after gastrointestinal digestion", FOOD RESEARCH INTERNATIONAL, vol. 105, 1 March 2018 (2018-03-01), AMSTERDAM, NL, pages 1003 - 1010, XP055821803, ISSN: 0963-9969, DOI: 10.1016/j.foodres.2017.12.038
- TENORE GIAN CARLO ET AL: "Antioxidant peptides from "Mozzarella di Bufala Campana DOP" after simulated gastrointestinal digestion: In vitro intestinal protection, bioavailability, and anti-haemolytic capacity", JOURNAL OF FUNCTIONAL FOODS, vol. 15, 1 May 2015 (2015-05-01), NL, pages 365 - 375, XP055821804, ISSN: 1756-4646, DOI: 10.1016/j.jff.2015.03.048
- ARA� JO DALINE F S ET AL: "Intestinal anti-inflammatory effects of goat whey on DNBS-induced colitis in mice", 28 September 2017 (2017-09-28), XP055821806, Retrieved from the Internet <URL:https://journals.plos.org/plosone/article?id=10.1371/journal.pone.0185382> [retrieved on 20210706]
- JAYA BENJAMIN ET AL: "Glutamine and Whey Protein Improve Intestinal Permeability and Morphology in Patients with Crohn’s Disease: A Randomized Controlled Trial", DIGESTIVE DISEASES AND SCIENCES, KLUWER ACADEMIC PUBLISHERS-PLENUM PUBLISHERS, NE, vol. 57, no. 4, 26 October 2011 (2011-10-26), pages 1000 - 1012, XP035031514, ISSN: 1573-2568, DOI: 10.1007/S10620-011-1947-9


(52) Cooperative Patent Classification (CPC): (Cont.)

C-Sets
A23V 2002/00, A23V 2200/32, A23V 2250/54252

## Description

The present invention relates to a nutraceutical composition comprising buffalo milk whey, preferably buffalo from the region of Campania, and its use for the reduction of intestinal permeability.

STATE OF ART

Buffalo milk is a very ancient product, typical of tropical and subtropical countries, such as Southeast Asia. Buffalo mozzarella is the most famous and most widely consumed buffalo milk product in Southern Italy. In particular, the so-called "Mozzarella di Bufala Campana" (MBC) [Buffalo Mozzarella of the region of Campania] is listed as a product with protected designation of origin (PDO) by the European Council, and nine provinces of Southern Italy are identified as the only production sites of the so-called "Mozzarella di Bufala Campana. DOP "or" MBC ". For its preparation, a particular production process is indicated which involves exclusively fresh whole buffalo milk from the Italian Mediterranean breed, characterized by a specific composition in macronutrients and with specific chemical-physical and organoleptic properties of the final product.

The milk from this unique animal has in nature the following chemical and physical composition. The percentage quantities provided hereunder are intended as a percentage (%) by weight, with respect to the total weight of the milk. Alternatively, the quantities may for some substances be in milligrams, per 100 grams of milk.

Water: 81.5%
Dry matter: 18.5%
Lean residue: 10.3%
Casein: 3.6%
Lactalbumin and lactoglobulin: 0.7%
Total nitrogenous substances: 4.3%
Fat: 7.2-9.6%
Lactose: 5%
Ash: 0.8%
Calcium: 180-240 mg, on 100 grams of milk
Phosphorus: 120-140 mg on 100 grams of milk
Ratio: Ca / P 1.61
Sodium: 40 mg per 100 grams of milk
pH: 6.5 / 6.7
Specific weight: 1.033 Kg/liter
Fat globule diameter: 1.0-6.0 microns

The physico-chemical characteristics in % of Mozzarella di Bufala Campana PDO are the following:

Humidity: 59% -64% in any case not higher than 65%
Dry residue: 35.8% -40.8%
Fat: 21% -26%
Protein: 12% -16%
Ash: 1.5% -1.8%
Lactose: 0.3%
Galactose: 0.2%
Lactic acid: 0.4%

The above percentage values are considered as percentage (%) by weight, with respect to the total weight of the Buffalo Mozzarella of Campania.

The organoleptic characteristics of MBC can be summarized as follows:

External appearance: porcelain white colour, very thin crust of about one millimeter with a smooth surface, never slimy or flaky.
Paste: structure with thin leaves, slightly elastic in the first eight to ten hours after production and packaging, subsequently tending to become softer; free from defects such as holes, caused by gaseous or anomalous fermentations; absence of preservatives, inhibitors and dyes; when cut there is draining in the form of a slight whitish, greasy serosity, with the scent of lactic ferments.
Taste: characteristic and delicate.

The dairy industry generates high quantities of whey, corresponding to the liquid fraction that remains after coagulation of milk and elimination of casein during cheese production, both from cheese and casein production plants.

In particular, whey is the liquid part of the milk that separates from the curd (obtained from the mixture of rennet and heated milk) during cheese making: the addition of rennet serves to cause the coagulation of the casein contained in the milk. Whey has fewer calories than milk, 27 calories per 100 grams of product, and also contains the following components expressed in grams per 100 grams of product, i.e. approximately:

- lactose: 4g/100g
- proteins: 1.10g/100g
- mineral salts: 0.5g./100g
- lipids: 1.2g/100g
- galactose: 0.3g/100g.

It is important to consider that there are different types of serums that can be obtained depending on the waiting times and the method used for curdling the milk and pouring it, two different serums can therefore have very different characteristics.

Over time, whey has been considered a waste product from cheese making, the most important by-product of the dairy industry not only for its high organic content, but also for its high volume. The perception of whey as a waste product however has changed with the recent discovery of its functional and bioactive properties; despite its polluting potential, it should, in fact, be considered a dairy by-product with nutritional, functional and bioactive properties, which represents about 85-90% of the milk volume and retains about 55% of the milk nutrients. Whey is currently used in the food industry especially for producing ricotta and as an additive in other food and pastry products.

In addition, whey and hydrolyzed proteins deriving from whey are now used in the sports industry, for products aimed at maintaining muscle tone and development and are considered ideal for all sports training goals. There are in fact numerous products on the market directed towards the sports sector, for example in the form of supplements such as drinks or powders to be diluted (body-building).

The bioactive properties of milk and dairy products are largely attributed to protein fragments, called peptides, which are released during gastrointestinal digestion. These peptides are inactive within the parent protein sequence and can be released by digestive enzymes during gastrointestinal transit, or by fermentation or maturation during food processing. The hydrolysis of whey proteins generates bioactive peptides, which are known to have physiological effects *in vivo,* such as an antioxidant, antimicrobial, antihypertensive and antidiabetic activity, in addition to an immunomodulatory, anti-cancer, opioid and hypocholesterolemic action, with a consequent beneficial effect on the cardiovascular, gastrointestinal, immune and nervous system.

Whey, and in particular the whey obtained from the processing of buffalo mozzarella, should not be confused with colostrum. Colostrum is produced by the secretion of a mammal's breast in the first few days after giving birth and is not considered to be real milk. Whey, on the other hand, is a substance that comes from dairy processing, in particular from the processing of mozzarella such as buffalo mozzarella from which it is separated before the spinning phase of the same.

The intestine represents the last portion of our digestive system and is also defined as the second brain, thanks to the presence of a real nervous system located in the thickness of its wall. It is the largest area of the body (about 7 meters long), and is the seat of the body's most important immune station as it represents the main interface for the passage from the external to the internal environment of the organism.

The wall of the intestinal lumen is organized as a multi-layered system which has the task of

- preventing bacterial adhesion,
- regulating the para-cellular diffusion towards the underlying host tissues, and
- discriminating between commensal micro-organisms (i.e. those microorganisms that do not cause damage) and pathogenic microorganisms (i.e. harmful microorganisms), organizing immunological tolerance towards commensal micro-organisms and immune response towards pathogenic microorganisms.

The surface barrier begins from the resident macrobiota (or intestinal flora) that competes with pathogens for gaining space and energy resources, processing the molecules necessary for mucosal integrity and modulating the immunological behaviour of the deep barrier. The next level is represented by the mucus layer, which separates the endoluminal contents of the intestine from the innermost layers and contains antimicrobial products and secretory IgAs (immunoglobulins A). Below the mucus there is a monolayer of epithelial cells, the enterocytes, tightly joined together by tight junctions, which allow a physical barrier to be formed effective in absorbing nutrients, but equally effective in preventing the majority of some of the harmful molecules and larger germs from passing from inside the intestine into the bloodstream and thus potentially causing various problems.

These defensive layers form the intestinal barrier.

The intestine is therefore located at the interface between the organism and its luminal environment and represents a critical defense barrier against toxic luminal agents. Consequently, in addition to being exposed to luminal nutrients, the intestinal mucosa is constantly threatened by oxidants, mutagens and carcinogens of food origin, and also by endogenous agents. The intestinal mucosa therefore exerts two apparently opposite functions: firstly, it allows a peaceful coexistence with intestinal symbiotics without causing chronic inflammation and, secondly, it provides an inflammatory and defensive response, measured on the basis of the "threat" of pathogen agents. It is a complex multilayer system consisting of an external "physical" barrier and an internal "functional" immunological barrier.

In particular, the intestine contains numerous elements with toxic properties such as, for example, fragments of bacterial cell walls, bacterial antigens or food antigens and this is why it must be capable of offering an effective barrier against the excessive absorption of the same which would cause significant damage..

When this barrier mechanism is ineffective, the protective function is impaired and these toxic elements/products pass into the bloodstream in excessive quantities, causing sensitization of the immune system.

In principle, the disorders deriving from these alterations can be localized only in the intestine, already causing even serious disorders or pathologies, but in the long term they can spread to other organs also belonging to districts far from the intestine. Disorders or pathologies originating in the intestine can, for example, be acute and chronic intestinal inflammation, digestive disorders, food intolerances but also migraines, type I diabetes, multiple sclerosis up to mental illnesses. The intestinal protective barrier is therefore of great importance for having and maintaining a healthy organism.

This barrier mechanism is regulated by intestinal permeability (IP) and the cooperation of these barriers ("physical" and "functional") allows a balanced permeability to be maintained.

The lactulose/mannitol ratio allows the permeability of the intestinal wall and its absorption capacity to be evaluated. The analysis is carried out through the collection of urine, and the detection of the lactulose/mannitol ratio in the urine itself. Mannitol and lactulose are two inert sugars, not metabolized by the intestine, and therefore the absorbed portion of these two sugars is completely excreted in the urine within six hours. In a healthy intestine, with an intestinal barrier in equilibrium, the absorption rate of mannitol is 14%. Lower values indicate impaired intestinal absorption. Lactulose, on the contrary, under physiological conditions and with an intestinal barrier in equilibrium, is absorbed to a very low extent, less than 1%. Higher values occur under conditions of impaired intestinal absorption.

The increase in intestinal permeability leads to a passive absorption of substances normally excluded, through actual breaches open in the intestinal mucosa, breaches through which proteins with a strong antigenic potential, toxins and pathogens can penetrate into the body. In this way, a multitude of clinical disorders are triggered up to even serious intestinal pathologies.

A dysregulation of intestinal permeability, for example, leads to:

- hyper-activation of the intestinal immune system which feeds local chronic inflammation which, in turn, gives rise to increased permeability; this creates a vicious circle that promotes further deterioration;
- entry into the bloodstream of immunogenic substances capable of triggering an immune response even in organs distant from the intestine, triggering inflammatory and/or autoimmune phenomena.

One of the intestinal diseases known to be caused by increased intestinal permeability is the so-called *"Leaky Gut Syndrome".* Among possible causes of an increase in permeability, which leads to the Leaky Gut Syndrome, there are incorrect eating habits, alcohol abuse, the use in considerable doses of certain categories of drugs (for example, antibiotic therapies or prolonged cortisone), environmental pollutants and stress. Various symptoms are attributed to this condition such as, for example, intestinal disorders (irritable bowel, constipation, dysentery), intolerances, allergies, dermatitis, thyroid disorders (hypothyroidism), joint and muscle pain, chronic fatigue or, on the contrary, hyperactivity.

Impaired barrier function is also one of the key events in the pathogenesis of Leaky Gut Syndrome known as Irritable Bowel Syndrome (IBS).

Irritable bowel syndrome (IBS) is a condition that manifests itself with varying intensity through multiple symptoms including abdominal pain and discomfort associated with diarrhea and/or constipation or both alternating disorders.

It can be defined as a chronic and recurrent disorder of the functions of the gastrointestinal system, mainly affecting the colon and small intestine with alterations in the motor functions, painful sensitivity and fluid secretion. These activities of alterations in the motor functions (motility, sensitivity and secretion) are regulated by the brain. As indicated above, the intestine is now recognized as the second brain of our body and for this reason irritable bowel syndrome is often also called a brain-intestinal axis disorder.

Abdominal pain or discomfort are the main symptoms of IB S. There are many causes of abdominal pain, but in IBS it is associated with changes in bowel emptying that are manifested with diarrhea and/or constipation.

People suffering from IBS may have predominantly diarrhea, or predominantly constipation or alternate periods of diarrhea with periods of constipation. The symptoms can change over time and from person to person, there may be periods with very intense symptoms, and also periods when the symptoms subside or disappear altogether.

IBS appears in three main forms, one characterized by predominant diarrhea (IBS-D - D: Diarrhea), more common in men; one characterized by constipation (IBS-C - C: Constipation), more common in women and not to be confused with other chronic inflammatory diseases (such as Crohn's disease) and another, less common, which alternates periods of constipation with periods of diarrhea (IBS-A - A: Alternate).

In addition, an altered intestinal barrier with an imbalance in the permeability of the wall can not only be a trigger for various intestinal dysfunctions, such as IBS mentioned above, but can also lead to chronic intestinal inflammation, up to Crohn's disease and ulcerative colitis.

Ulcerative colitis is a chronic inflammatory and ulcerative disease that originates in the mucous membrane of the colon and is most often characterized by bloody diarrhea. This type of colitis usually begins in the rectum, and then extends to the entire colon with very painful symptoms. The long-term risk of colon cancer is high with respect to patients suffering from this type of chronic inflammation.

Crohn's disease is also characterized by chronic inflammation of the intestine, which can affect the entire gastrointestinal tract, from the mouth to the anus. In about 90% of cases, the disease mostly affects the last part of the small intestine (ileum) and the colon. The disease is characterized by intestinal ulcers, often alternating with sections of healthy intestine, and, if not treated properly, can lead to complications such as strictures or fistulas that may require surgery.

Crohn's disease can occur in various ways depending on the intestinal localizations. The most frequent symptoms are chronic diarrhea (i.e. that persists for more than 4 weeks), often nocturnal, associated with abdominal pain and cramps, sometimes with bleeding mixed with the feces, and with fever that arises in the evening, or with joint pain, or with other non-intestinal manifestations. There can often be a significant weight loss. Sometimes, it can be manifest at the anal level with fistulas or collections of pus (abscesses). Crohn's disease and ulcerative colitis are identified as IBD (Inflammatory Bowel Disease).

Ulcerative rectal colitis is again a chronic inflammatory disease that primarily affects the rectum and can involve part or all of the colon. The causes of this inflammation are still unknown. The main clinical symptoms are diarrhea, often with blood and mucus, and abdominal pain. In many cases extra-intestinal symptoms may be present, such as joint pain and dermatological manifestations. In some subjects the symptoms are so violent with numerous discharges of liquid feces with blood, dehydration and fever, that urgent hospitalization is necessary for administering vein therapies and keeping the patient under close medical supervision.

This type of chronic inflammation of the intestine negatively affects the life of anyone affected by it, with a significant reduction in the quality of life. They are considered disabling diseases or disorders.

The types of treatment currently known are based on the use of drugs such as 5-aminosalicylic acid (for example mesalazine), corticosteroids (for example budesonide), immunomodulators, biological drugs, antibiotics, sometimes resorting to surgery.

The repeated and chronic administration of this kind of drugs is often associated with side-effects, sensitization to the drugs themselves with a reduction in the efficacy, dependence or even resistance.

More specifically, the following documents of prior art are known:

- the article of De Simone Carmela et al "Characterisation and cytomodulatory properties of peptidesfrom Mozzarella di Bufala Campana cheese whey Bioactive peptides in mozzarella di Bufala whey" (Journal of peptide Science, vol 15, no 3, 26 November 2008, pages 251-258, XP055821798, ISSN. 1075-2617, DOI: 10 1002/psc.1093) that discloses buffalo skimmed whey samples (BSW, BWW and BS) which are lyophilized (after ultra-filtration), the whey samples being obtained from the processing of buffalo mozzarella;
- the article of Basilicata Manuela et al.: "Antioxidant Properties of Buffalo-Milk Dairy Products A [beta]-Lg Peptide Released after Gastrointestinal Digestion of Buffalo Ricotta Cheese Reduces Oxidative Stress in Intestinal Epithelial Cells" (International Journal of Molecular Sciences, vol 19, no 7,4 July 2018, page 1955, XP055821799, DOI 10 3390/ijms19071955) that investigates the release after an in vitro gastrointestinal digestion of potential antioxidant peptides from buffalo-milk dairy products;
- the article of De Simone Carmela et al. "Peptides from water buffalo cheese whey induced senescence cell death via ceramide secretion in human colon adenocarcinoma cell line" (MOLECULAR NUTRITION & FOOD RESEARCH, vol 55, no. 2, 19 August 2010, pages 229-238, XP055821566, DE ISSN: 1613-4125, DOI: 10.1002/mnfr 201000074) that discloses the effects of 5 BWW sub-fractions on: (i) mitochondrial oxidative stress and expression of Hsp 70 and Hsp 90; (ii) cell cycle and differentiation; and (iii) production of ceramides and senescence. Said BWW was produced during "Mozzarella di Bufala Campana Protected Denomination of Origin" manufacturing;
- the article of Tenore Gian et al. "Intestinal Anti-Inflammatory Effect of a Peptide Derived from Gastrointestinal Digestion of Buffalo (Bubalus buballs) Mozzarella Cheese" (Nutrients, vol 11, no 3, 13 March 2019 (2019-03-13), page 610, XP055821800, 001 10 3390/nu11030610) that discloses MBCP, a peptide isolated from the buffalo milk after in vitro digestion of Mozzarella di Bufala Campana DOP, which possesses a good stability to brush border exopeptidases and a high bioavailability; and
- DATABASE GNPD [Online] MINTEL; 31 October 2006, anonymous. "Diet Capsules", XP055821813, Database accession no 602320, that discloses a nutritional composition comprising whey in the form of a capsule.

The necessity is therefore felt for finding a product capable of regulating intestinal permeability, which can be effective in the treatment of chronic inflammatory bowel disorders, such as ulcerative colitis, ulcerative rectum-colitis and Crohn's disease, with no side-effects.

It has now been surprisingly found that a composition comprising buffalo milk whey, preferably buffalo from the region of Campania, is capable of reducing intestinal permeability and restoring the barrier system of the intestine to a correct equilibrium.

DESCRIPTION

The present invention relates to a nutraceutical composition according to the appended claims set.

The present invention further relates to a nutraceutical composition comprising or consisting of buffalo milk whey, preferably buffalo of the Italian Mediterranean breed, for use in the treatment of chronic inflammatory diseases of the intestine, preferably for the treatment of ulcerative colitis, ulcerative rectal colitis, Crohn's disease, irritable bowel syndrome (IBS) and/or Leaky Gut Syndrome.

The whey of buffalo milk, preferably buffalo of the Italian Mediterranean breed, according to the present invention is obtained by processing buffalo mozzarella from which it is preferably separated before the spinning phase of the same.

The buffalo milk whey according to the present invention is mainly composed of biologically active peptides and its analytical composition corresponds to a mixture of caseins, preferably alpha-casein, beta-casein and kappa-casein. More specifically, the alpha-casein is S1 alpha-casein. The blend of caseins representing biologically active peptides is indicated in more detail in Table 1 hereunder:

Table 1

| Alpha Casein S1 | Beta Casein | Kappa casein |
|---|---|---|
| NH2-FVAPFPEVF-COOH | NH2-DELQDKIHPF-COOH | NH2-MAIPPKKNQDKTEIPTINT-COOH |
| NH2-VAPFPEVF-COOH | NH2-DELQDKI HPFAQT-COOH | NH2-MAIPPKKN-COOH |
| NH2-FVAPFPEVFGKEKVNE-COOH | NH2-DELQDKIHPFAQTQ-COOH | NH2-INNQFIPYPYYAKPAA-COOH |
| | NH2-DELQDKIHPFAQTQSLVYPFPGPIP-COOH | NH2-MAIPPKKNQ-COOH |
| NH2-YQLDAYPSGAW-COOH | | NH2-SRYPSYGLN-COOH |
| NH2-DAYPSGAW-COOH | NH2-LVYPFPGPIP-COOH | NH2-SRYPSYGINY-COOH |
| NH2-FVAPFPEV-COOH | NH2-GVSKVKEAMAPK-COOH | NH2-VISRYPSY-COOH |
| NH2-QKEPMIGVNQEL-COOH | NH2-EMPFPKYPVEPF-COOH | NH2-YYQQKPVA-COOH |
| NH2-SDIPNPIGSENSGKTTMPL-COOH | NH2-LTDVENLHIPIPL-COOH | NH2-MAIPPKKN-COOH |
| | NH2-LVYPFPGPIP-COOH | NH2-MAIPPKKNQDKTEIPTINT-COOH |
| | NH2-LVYPFPGPIP-COOH | NH2-INNQFLPYPYYAKPAAVRSPAQIL-COOH |
| | NH2-LYQEPVIGPVRGPFPIIV-COOH | |
| | NH2-QEPVIGPVRGPFPI-COOH | NH2-SRYPSYGLN-COOH |
| | NH2-SLSQSKVLPVPQKAVPYPQRDMPIQ-COOH | NH2-SRYPSYGLNY-COOH |
| | | NH2-VLSRYPSY-COOH |
| | NH2-<br>SLSQSKVLPVPQKAVPYPQRDMPIQA-COOH | NH2-VLSRYPSY-COOH<br>NH2-MAIPPKKNQ-COOH |
| | | NH2-INNQFLPYPYYAKPAAVRSPAQILQW-COOH |
| | NH2-TDVENIHLP-COOH | |
| | NH2-TDVENIHLPL-COOH | |
| | NH2-TDVENLHIPIPLL-COOH | NH2-MAIPPKKNQDKTEIPTINT-COOH |
| | NH2-TDVENLHIPIPLLQS-COOH | NH2-INNQFIPYPYYAKPAA-COOH |
| | NH2-TLTDVENIHLP-COOH | NH2-YYQQKPVA-COOH |
| | NH2-YQEPVIGPVRGPFP-COOH | |
| | NH2-YQEPVIGPVRGPFPI-COOH | |
| | NH2-YYQQKPVA-COOH | |
| | NH2-SLSQSKVLPVPQKAVPYPQRDMPIQ-COOH<br><br>NH2-SLSQSKVLPVPQKAVPYPQRDMPIQAFL-COOH<br><br><br>NH2-TLTDVENLHLPLPL-COOH<br>NH2-YQEPVLGPVRGPFPI-COOH | |

(continued)

| Alpha Casein S1 | Beta Casein | Kappa casein |
|---|---|---|
| | NH2-EELNVPGEIVE-COOH | |
| | NH2-LTDVENLHLPLPL-COOH | |
| | NH2-LYQEPVLGPVRGPFPIIV-COOH | |
| | NH2-SLPQNIPPLTQTPVVVPPF-COOH | |
| | NH2-SLSQSKVLPVPQ-COOH | |
| | NH2-SLSQSKVLPVPQKAVPYPQRDMPIQA-COOH | |
| | | |
| | NH2-TDVENLHLP-COOH | |
| | NH2-TDVENLHLPL-COOH | |
| | NH2-TDVENLHLPLPL-COOH | |
| | NH2-TDVENLHLPLPLL-COOH | |
| | NH2-TLTDVENLHLP-COOH | |
| | NH2-YQEPVLGPVRGPF-COOH | |
| | NH2-YQEPVLGPVRGPFP-COOH | |
| | NH2-YQEPVLGPVRGPFPII-COOH | |
| | NH2-LLYQEPVLGPVRGPFPIIV-COOH | |
| | NH2-LVYPFPGPIP-COOH | |
| | NH2-QEPVLGPVRGPFPI-COOH | |
| | NH2-QEPVLGPVRGPFPI-COOH | |
| | NH2-SQSKVLPVPQKAVPYPQRDMPIQAFL-COOH | |

The term "comprises" means that the nutraceutical composition may contain other substances or other components such as, for example, excipients or also other active substances.

The term "consists of" means that the nutraceutical composition contains only whey.

Said buffalo milk whey, preferably buffalo from the region of Campania, according to the present invention is used in lyophilized form, called *"lioMBCP".*

According to an embodiment of the present invention, the buffalo milk whey is subjected to lyophilization through known lyophilization processes, with a yield of about 10%.

The nutraceutical composition of the present invention is formulated in the form of a capsule.

Said oral solid form according to the present invention can also be with immediate release or with controlled release, in gastro-resistant form. According to the present invention is a gastro-resistant capsule.

According to the present invention, the nutraceutical composition comprises buffalo milk whey, lyophilized Italian Mediterranean buffalo whey, formulated in the form of a gastro-resistant capsule.

The capsule form according to the present invention can be produced through a hard capsule or a soft capsule, preferably a hard capsule. The term gastro-resistant according to the present invention means that the composition is resistant to gastric fluid, and passes through the stomach unaltered, releasing the contents at the level of the intestinal fluid where it exerts its action. The site-specific release of the nutraceutical composition of the present invention is essential for achieving the desired effect, so that the buffalo whey is essentially released only at the site of action - in the intestine - without being dispersed in districts not affected by the permeability dysfunction. In this way, it is possible to maximize the effectiveness of the bioactive components of buffalo whey.

According to the present invention, gastro-resistance is a characteristic of the capsule form where the gastro-resistance is given by the material of which the capsule itself is made, which guarantees passing the stomach in an unaltered form with the release of its contents into the intestinal fluid where it exerts its action.

The whey of buffalo milk, preferably buffalo of the Italian Mediterranean breed, is contained in the nutraceutical composition of the present invention in a quantity equal to 500 mg.

According to an embodiment of the present invention, the total weight of the nutraceutical composition is intended as the weight of the buffalo milk whey alone when no other component is contained in the composition. According to a preferred embodiment, the composition of the present invention consists of buffalo whey, i.e. it comprises buffalo whey alone and consequently the total weight of the composition corresponds to the weight given by the sole quantity of buffalo whey. The buffalo whey of the present invention is in lyophilized form.

An advantage of the nutraceutical composition as described above is to rebalance the barrier function of the intestinal mucosa, made more permeable to toxic agents for various causes such as, for example, incorrect eating habits, alcohol abuse, use in considerable doses of some categories of drugs (for example, prolonged antibiotic or cortisone therapies) or environmental pollutants and/or stress.

The nutraceutical composition according to the invention acts effectively on the intra- and inter-cellular spaces that characterize the mucosa of the intestinal wall, reducing the spaces between cells and consolidating the tight junctions between the cells of the mucosa. In this way, the nutraceutical composition of the present invention acts by reducing intestinal permeability and the consequent possibility of toxic agents passing through the intestinal barrier. In this way, the efficacy of the nutraceutical composition of the present invention leads to an improvement in the painful symptoms of patients suffering from inflammatory intestinal diseases and disorders, such as ulcerative colitis, ulcerative rectal colitis, Crohn's disease or IBS in its various forms.

The nutraceutical composition comprising buffalo whey, preferably buffalo of the Italian Mediterranean breed, of the present invention is also intended for use in the treatment of chronic inflammatory bowel diseases, preferably for the treatment of ulcerative colitis, ulcerative rectal colitis, Crohn's disease, irritable bowel syndrome (IBS) and/or Leaky Gut Syndrome.

According to a preferred embodiment, the nutraceutical composition of the present invention is preferably administered according to a regimen of 4 capsules, for a period of time ranging from 4 to 8 weeks.

Some formulation examples are provided hereunder for a better understanding of the present invention.

EXAMPLES

Example 1

Preparation of gastro-resistant capsules

500 mg gastro-resistant capsules were selected, whose heads and bodies are composed of titanium dioxide 1.9% (equal to 2.6 mg), gellan gum 5.0% (equal to 5.5 mg), hypromellose sufficient for reaching 100% (equal to 120.9 mg). The lyophilized whey of Campania buffalo is inserted inside the capsules through a suitable mixer, in an amount equal to 500 mg for each capsule. A single capsule, before being filled with 500 mg of lyophilized buffalo whey (i.e., in its empty form), has a weight of approximately 130 mg.

Example 2 (not according the invention)

Preparation of sachets

The lyophilized Campania buffalo whey was prepared so as to fill a sachet to ensure a total content of 1g.
For better illustrating the invention, an experimental part is provided in this Example 3 to be considered as a support of the invention.

Example 3

EXPERIMENTAL PART

Randomized, placebo-controlled, single-centre, double-blind, intervention study with a nutraceutical preparation called MBCP based on peptides from whey waste from cheese making.

OBJECTIVES:

The purpose of the study is to evaluate whether the administration of a composition according to the present invention, which includes buffalo milk whey, preferably of the Italian Mediterranean breed, lyophilized in a gastro-resistant capsule prepared for a duration of 8 weeks, can allow a decrease in intestinal permeability, assessed by measurements of the lactulose/mannitol ratio in the urine of the subjects.

POPULATION BEING STUDIED:

Subjects with varying degrees of chronic intestinal inflammation, irritable bowel syndrome, Crohn's disease, ulcerative rectum-colitis.

SAMPLE SIZE:

80 subjects to be divided into two groups (40 subjects per group). The sample size was calculated using Power Analysis.

Materials and methods

Formulation of the nutraceutical product

The waste whey from MBCP cheese-making was supplied by the dairy Cilento S.p.A. at the NutraPharmaLabs Laboratories of the Department of Pharmacy of Naples (UNINA). The serum was subjected by UNINA to lyophilization (lioMBCP), with a yield of about 10%. UNINA laboratory analyses of lioMBCP revealed a high content of biologically active peptides. LioMBCP was used by UNINA for formulating a nutraceutical product based on gastro-resistant capsules (lioMBCP content: 500 mg each).

Characterization of the serum peptides

A sample of lioMBCP was subjected to analysis by ultra-performance liquid chromatography coupled with mass spectrometry (UPLC ESI-MS/MS); an ACQUITY UPLC system (Waters Corporation, Milford, MA, USA) was used, in combination with a time-of-flight mass spectrometer (UPLC-ESI Q-TOF MS) (Waters Corporation). An aliquot of lioMBCP was solubilized in the mobile phase and 5 $\mu$L of peptide solution were charged onto an ACQUITY BEH C18 column (100 mm x 2.1 mm, 1.7 $\mu$m) (Waters Corporation). The elution conditions consisted of acetic acid (eluent A) and methanol (eluent B) with a flow-rate of 0.3 mL/min: 0-3 min, 5% B; 3-15 min, 5%-30% B; 15-17 min, 30% B. After molecular mass determination, the peptides were automatically selected for fragmentation, and sequence information was obtained by tandem mass spectrometry analysis. MS/MS analyses were carried out in positive electrospray ionization mode using CID. The collision energy was selected from 10 to 35 eV. As collision gas, Argon was introduced at a pressure of 10 psi. Peptide sequencing was acquired within the range of 50-2000 m/z using Biolynx software.
As indicated above, the characterization of the peptides corresponds to Table 1 hereunder:

Table 1

| Alpha Casein S1 | Beta Casein | Kappa casein |
|---|---|---|
| NH2-FVAPFPEVF-COOH<br>NH2-VAPFPEVF-COOH<br>NH2-FVAPFPEVFGKEKVNE-COOH<br>NH2-YQLDAYPSGAW-COOH<br>NH2-DAYPSGAW-COOH<br>NH2-FVAPFPEV-COOH<br>NH2-QKEPMIGVNQEL-COOH<br>NH2-SDIPNPIGSENSGKTTMPL-COOH | NH2-DELQDKIHPF-COOH<br>NH2-DELQDKI HPFAQT-COOH<br>NH2-DELQDKIHPFAQTQ-COOH<br>NH2-DELQDKIHPFAQTQSLVYPFPGPIP-COOH<br>NH2-LVYPFPGPIP-COOH<br>NH2-GVSKVKEAMAPK-COOH<br>NH2-EMPFPKYPVEPF-COOH<br>NH2-LTDVENLHIPIPL-COOH<br>NH2-LVYPFPGPIP-COOH<br>NH2-LVYPFPGPIP-COOH<br>NH2-LYQEPVIGPVRGPFPIIV-COOH<br>NH2-QEPVIGPVRGPFPI-COOH<br>NH2-SLSQSKVLPVPQKAVPYPQRDMPIQ-COOH<br>NH2-SLSQSKVLPVPQKAVPYPQRDMPIQA-COOH | NH2-MAIPPKKNQDKTEIPTINT-COOH<br>NH2-MAIPPKKN-COOH<br>NH2-INNQFIPYPYYAKPAA-COOH<br>NH2-MAIPPKKNQ-COOH<br>NH2-SRYPSYGLN-COOH<br>NH2-SRYPSYGINY-COOH<br>NH2-VISRYPSY-COOH<br>NH2-YYQQKPVA-COOH<br>NH2-MAIPPKKN-COOH<br>NH2-MAIPPKKNQDKTEIPTINT-COOH<br>NH2-INNQFLPYPYYAKPAAVRSPAQIL-COOH<br>NH2-SRYPSYGLN-COOH<br>NH2-SRYPSYGLNY-COOH<br>NH2-VLSRYPSY-COOH<br>NH2-VLSRYPSY-COOH<br>NH2-MAIPPKKNQ-COOH |
| | NH2-TDVENIHLP-COOH<br>NH2-TDVENIHLPL-COOH<br>NH2-TDVENLHIPIPLL-COOH<br>NH2-TDVENLHIPIPLLQS-COOH<br>NH2-TLTDVENIHLP-COOH<br>NH2-YQEPVIGPVRGPFP-COOH<br>NH2-YQEPVIGPVRGPFPI-COOH<br>NH2-YYQQKPVA-COOH<br>NH2-SLSQSKVLPVPQKAVPYPQRDMPIQ-COOH<br>NH2-SLSQSKVLPVPQKAVPYPQRDMPIQAFL-COOH<br>NH2-TLTDVENLHLPLPL-COOH<br>NH2-YQEPVLGPVRGPFPI-COOH | NH2-INNQFLPYPYYAKPAAVRSPAQILQW-COOH<br>NH2-MAIPPKKNQDKTEIPTINT-COOH<br>NH2-INNQFIPYPYYAKPAA-COOH<br>NH2-YYQQKPVA-COOH |

(continued)

| Alpha Casein S1 | Beta Casein | Kappa casein |
|---|---|---|
| | NH2-EELNVPGEIVE-COOH<br>NH2-LTDVENLHLPLPL-COOH<br>NH2-LYQEPVLGPVRGPFPIIV-COOH<br>NH2-SLPQNIPPLTQTPVVVPPF-COOH<br>NH2-SLSQSKVLPVPQ-COOH<br>NH2-SLSQSKVLPVPQKAVPYPQRDMPIQA-COOH<br><br>NH2-TDVENLHLP-COOH<br>NH2-TDVENLHLPL-COOH<br>NH2-TDVENLHLPLPL-COOH<br>NH2-TDVENLHLPLPLL-COOH<br>NH2-TLTDVENLHLP-COOH<br>NH2-YQEPVLGPVRGPF-COOH<br>NH2-YQEPVLGPVRGPFP-COOH<br>NH2-YQEPVLGPVRGPFPII-COOH<br>NH2-LLYQEPVLGPVRGPFPIIV-COOH<br>NH2-LVYPFPGPIP-COOH<br>NH2-QEPVLGPVRGPFPI-COOH<br>NH2-QEPVLGPVRGPFPI-COOH<br>NH2-SQSKVLPVPQKAVPYPQRDMPIQAFL-COOH | |

Clinical study

Study participants were recruited from the patient database of the Samnium Medica Cooperative, Benevento, Italy. Subjects of both sexes, aged between 18 and 75 years, with varying degrees of chronic intestinal inflammation, irritable bowel syndrome, Crohn's disease, ulcerative rectal colitis were enrolled in July 2018. The exclusion criteria adopted were: smoking, obesity (BMI> 30 kg/$m^2$), diabetes, liver disease, kidney disease, heart disease, family history of chronic diseases, intense exercise (> 10 h/week), pregnant women, women suspected of being pregnant, women hoping to become pregnant, breastfeeding, birch pollen allergy, and blood donation in the three months prior to the study. The subjects received oral and written information regarding the study before giving their written consent. The protocol, the letter of intent from the volunteers and the synoptic document on the study were submitted to the Scientific Ethics Committee of the AO Rummo Hospital (Benevento, Italy). The study was approved by the committee and was performed in accordance with the 1964 Helsinki Declaration (revised in 2000). The subjects were asked to make records of whey intakes in the control table for the intervention study and to make side-effect records in the daily reports. The study was a randomized, double-blind, single-centre, placebo-controlled study. The duration of the study was 16 weeks. A number of 80 subjects were enrolled, randomly divided into two groups.

Evaluation criteria

After an initial run-in period, the subjects were asked to take 4 capsules of MBCP daily (500 mg each, 2 capsules at lunch and 2 capsules at dinner). This administration has a duration of 8 weeks. At the beginning of the study (T0), after 30 days (T1) and after 60 days (T2), the subjects took 300 mL of an aqueous solution consisting of 5 g of lactulose, 2 g of mannitol and 100 g of sucrose. This was followed by a sample of urine after about 5-6 hours. In addition, systolic and diastolic blood pressure was monitored. At the end of the dosing period, a follow-up period of 4 weeks was effected. The total duration of the study was 16 weeks (see Figure 1).

Protocol

The subjects were asked to take 4 capsules of lyophilized protein whey daily (500 mg each, 2 capsules at lunch and 2 capsules at dinner). This administration lasted 8 weeks. At the start of the study (T0), after 30 days (T30) and after 60 days (T60), the subjects took 50 mL of a 66.7% w/V aqueous solution of lactulose and 18% w/V of mannitol:

Lactulose: 10 g; 66.7% w/V
Mannitol: 2 g; 18% w/V
66.7 g: 100 mL = 10 g: x mL
x = 15 mL; volume of 66.7% solution of lactulose; 18 g: 100 mL = 2 g: x mL
x = 11.11 mL; volume of 18% solution of mannitol.

Calculating for a total of 50 mL of solution:

15 mL (lactulose): 100 mL = x mL (lactulose): 50 mL
x = 7.5 mL of lactulose
11.11 mL (mannitol): 100 mL = x mL (mannitol): 50 mL
x = 5.5 mL of mannitol.

A urine sample was then taken after about 5-6 hours. The systolic and diastolic blood pressure was also monitored. At the end of the administration period, a follow-up period of 4 weeks was carried out. All of the samples were stored at -80°C until analysis. Standardized telephone interviews were carried out every 5 days starting from the first clinical examination, in order to verify compliance and increase adherence to the protocol. **In** particular, these interviews reminded the patients to complete the intake checklist for the intervention study and record any treatment interruptions, or adverse events they might have experienced in the meantime (which were documented regularly on case forms during every telephone call and clinical examination). At every clinical examination, patients were required to complete three self-administered questionnaires on aspects of their quality of life and their diaries were controlled for completeness of the data and quality of the documentation.

Randomization

A total of 80 eligible patients (48 men and 32 women) were randomly assigned to two subgroups (each of 40 individuals). If a patient left the group before the intervention period, he/she was replaced by the next eligible patient enrolled. Covert allocation was effected by an Internet-based randomization program, stratified by study site. The list of random numbers was generated by an investigator with no clinical involvement in the study. Patients, doctors, laboratory technicians and trial staff (data analyst statisticians) were unaware of the treatment assignment.

Primary and secondary efficacy outcomes

The primary endpoints measured were changes in the degree of intestinal permeability. All untreated patient considerations were blindly assessed at the main investigation site.

The decision-taking process was performed on the basis of a consensus document (standard operating procedure not published).

Safety

Safety was determined through reports of adverse events, laboratory parameters regarding liver and kidney function, vital signs (blood pressure, pulse, height, weight and body mass index), physical and neurological tests. The safety was assessed for the entire treatment period on days 8, 35, 42 and 70, including adverse events occurring in the first three weeks after termination of the treatment.

Power-analysis

For the analysis of the proportions, the *effect-size* is estimated by the relative risk frequencies RR between the two experimental groups (e.g. in the primary study RR corresponds to the probability of receiving a drug treatment/probability of not receiving it). As no previous studies similar to this are available, the effect size assessments were conducted assuming the following scenarios:

- Risk in the placebo group: 99%, 90%, 80%
- Risk in the MBCP group: 70%, 50%, 30%

The RR values used for the estimation of the sample size deriving from it, are indicated in Table 2 hereunder.

Table 2:

| | 99% | 90% | 80% |
|---|---|---|---|
| 70% | 1.41 | 1.28 | 1.14 |
| 50% | 1.98 | 1.80 | 1.60 |
| 30% | 3.30 | 3.00 | 2.67 |

The sample size calculations were effected with three 1-β power values equal to 0.80, 0.95 and 0.99 and a significance level α = 0.05.

Table 3 hereunder shows the overall sample sizes for the various hypotheses formulated.

Table 3

| Risk in the placebo group | Risk in the MBCP group | RR (placebo/MBCP) | (1-P)=0.99 a = 0.05 | (1-P)=0.95 a = 0.05 | (1-P)=0.80 a = 0.05 |
|---|---|---|---|---|---|
| 99% | 30% | 3.30 | 30 | 24 | 18 |
| | 50% | 1.98 | 56 | 44 | 30 |
| | 70% | 1.41 | 118 | 88 | 60 |
| 90% | 30% | 3.00 | 46 | 36 | 26 |
| | 50% | 1.80 | 96 | 72 | 48 |
| | 70% | 1.28 | 304 | 222 | 144 |
| 80% | 30% | 2.67 | 70 | 54 | 36 |

(continued)

| Risk in the placebo group | Risk in the MBCP group | RR (placebo/MBCP) | (1-P)=0.99 a = 0.05 | (1-P)=0.95 a = 0.05 | (1-P)=0.80 a = 0.05 |
|---|---|---|---|---|---|
| | 50%<br>70% | 1.60<br>1.14 | 190<br>1408 | 80<br>1008 | 90<br>626 |

Based on what is indicated above, it is believed that an adequate number of patients could be equal to 80, of which 40 will be treated with MBCP and 40 with placebo, in a study in which it is assumed:

1) that 20% of the patients in the control group (placebo) recover spontaneously
2) that 50% of the patients in the group treated with MBCP recover thanks to the effect of the peptides, and
3) wherein the power of the study is 95%, indicating that 5 out of 100 cases can be estimated as false positives.

Statistics and methodology

During the process, it became apparent that dropouts and incomplete diary documentation resulted in missing data that could not be adequately handled by the expected robust comparison. In order to manage the structure of the missing data, a negative binomial was used, a model of generalized linear mixed effects (NB GLMM) which not only provides unbiased parameter estimates in the case of missing random data *"Missing at Random"* (MAR), but also provides reasonably stable results when the MAR intake is violated. Patients who did not provide diary data (which resulted in zero evaluable days) were excluded from the primary efficacy analysis based on MAR, according to an "all observed data approach" (White IR, Carpenter J, Horton NJ. Including all individuals is not enough: lessons for intention-to-treat analysis. Clin Trials 2012; 9: 396-407. DOI: 10.1177/1740774512450098"). This approach is statistically efficient without using multiple imputation techniques. The data recovered after treatment withdrawal from the randomized study was also included in the analysis. Unless otherwise indicated, all experimental results were expressed as the mean ± standard deviation (SD) of at least three replicates.

The statistical analysis of the data was effected via Student t-test or two-way ANOVA, followed by the Tukey-Kramer multiple comparison test for evaluating significant differences between a pair of media. The statistical heterogeneity was assessed using the Cochran test ($p < 0.1$). The I2 statistic was also calculated, and I2> 50% was considered as significant heterogeneity between the studies. If significant heterogeneity between the studies was demonstrated, a random-effect model was used. If not, the results were obtained from a fixed-effect model. The percentage change in the average and SD values was excluded when determining SD values for a result. The SD values were calculated from standard errors, 95% CI, p-value, or t-value if they were not available directly. The subgroup analyses, defined above, were carried out for examining possible sources of heterogeneity within these studies and they included health status, study design, type of intervention, duration, total dose of polyphenols, and Jadad score. The treatment effects were analyzed using PROC MIXED with treatment and period as fixed factors, subjects as random factors and baseline measurements as covariates and, defined as weighted average differences and 95% CI, and calculated for net changes in the parameters evaluated. The data that could not satisfy the criteria of variance homogeneity (Levenes test) and normal distribution (determined by an examination of the residual diagram and the Shapiro-Wilks test) even after the logarithmic transformation, were analyzed by a non-parametric test (Friedman). The significance level ($\alpha$ value) was 95% in all cases ($P < 0.05$).

Calibration curve

The calibration line is the line constructed with the least squares method, such as to minimize the sum of the squares of the residuals corresponding to all of the points.
The equation of this line is of the type y = a + bx, wherein:

- the intercept a is obtained from the equation: a = y - bx
- the angular coefficient b (regression coefficient), is given by the relation:

$$b = \frac{\Sigma(x_i - x_m)(y_i - y_m)}{\Sigma(x_i - x_m)^2}$$

Of which $x_i$ are all the values of the independent variable x and $y_i$ those of the dependent variable y. In order to establish to what extent the regression equation calculated with the least squares method can be used for finding a value of x knowing the value of y, a particular parameter is calculated, called determination coefficient:

$$R^2 = [\Sigma xy - (\Sigma x)(\Sigma y)/n]2/[\Sigma x2 - (\Sigma x)2/n][\Sigma y2 - (\Sigma y)2/n]$$

$R^2$ can have values ranging from 0 to 1, in this case, in order to be considered acceptable, it must generally not be less than 0.7.
If $R^2 = 1$ there is a perfect linear relationship between x and y, so that one and only one value of y corresponds to a given value of x.

The square root of the determination coefficient is the correlation coefficient: $r = \sqrt{R^2}$ wherein r can have values ranging from -1 to +1, in particular, a correlation coefficient r > 0.99 is considered as an indicator of linearity.
The calibration line is obtained by comparing the known concentrations, expressed in mg/mL, with the relative areas obtained by HPLC-MS/MS (see the lactulose line in Figure 2 and the mannitol line in Figure 3).
The construction of the calibration line therefore allows a quantitative analysis of the concentration of lactulose and mannitol present in the urine to be carried out, the peak area obtained by HPLC-MS/MS being known (Figure 2 and Figure 3, respectively).

Analysis set

The whole analysis set group included all randomized patients and patients who did not fail to meet an important entry criterion. Patients who did not provide primary efficacy data from efficacy analyses were excluded. The protocol set consisted of all patients who did not substantially deviate from the protocol and these had two characteristics: first of all, this group included patients for whom no major protocol violations had been detected (e.g., poor compliance, errors in 'assignment of the treatment); secondly, they had to be treated for at least 50 days starting from the day of the first intake (completion of a certain pre-specified minimum exposure to the treatment regimen). Patients who discontinued the study or treatment prematurely were therefore excluded from the protocol sample.

Involvement of the patient

No patient was involved in defining the research question or outcome measures, neither was he/she involved in the development of plans for the recruitment of participants or in the design and implementation of the study; there are, in fact, no plans for explicitly involving patients.
The final results were sent to all of the participants.

HPLC-MS / MS conditions

The analyses were effected on a Jasco Extrema LC-4000 system (Jasco Inc., Easton, MD) coupled with an Advion Expression mass spectrometer (Advion Inc., Ithaca, NY) equipped with an Electrospray (ESI) source. The mass spectra were recorded in negative SIM mode. The capillary voltage was set at -150 V, the spray voltage at 3 kV, the source voltage offset at -25 V and the capillary temperature at 300°C. The chromatographic separation was tested on the analytical Luna-NH2 column (150 × 4.6 mm, id. 3m, 100 A) and on the safety guard column, both supplied by Phenomenex (Torrance, CA, USA). The analyses were carried out at a flow-rate of 1 mL/min, with solvent A (ACN + 0.05% HCOOH) and solvent B (0.05% HCOOH). The elution was measured according to the following linear gradient: from 75% (A) to 40% (A) in 6 minutes. The urine injection volume was 10 μL and the column temperature was set at 30°C. For the quantitative analysis, standard curves of lactulose and mannitol (Sigma-Aldrich St. Louis, MO, USA) were prepared within a concentration range of 0.1-1.0 μg/μL with six different concentration levels and double injections for each level (Kubica et al. 2012). All of the data were collected and processed using JASCO ChromNAV (version 2.02.04) and Advion data express (4.0.13.8).

Results and discussion

Intestinal Permeability Assessment (lactulose/mannitol test)

Permeability assays generally involve the use of low-molecular-weight oligosaccharides, carbohydrates and/or polyalcohols such as mannitol MAN (~ 5-7 A), which freely pass through the mucosa through the aqueous pores on the cell membrane and are therefore absorbed by a "transcellular" route and disaccharides such as LAC lactulose (~ 10-12 A) which however use a "paracellular" route through the *"tight junctions"* between adjacent enterocytes.
The L/M test evaluates the permeability of the small intestine by measuring urinary excretion after oral administration of these sugars. As already mentioned, as lactulose is a large oligosaccharide usually not transported by the paracellular route, it can be adsorbed only in the case of loss of intercellular junctions unlike mannitol, a smaller molecule conceived for freely crossing the intestinal barrier regardless of the loss of the barrier function, and affected in the same way as large molecules, by pre- and post- mucosal factors such as gastric dilution, gastrointestinal motility, bacterial degradation and renal function. The ratio of the urinary concentration of both molecules (L/M ratio), measured after 5-6 hours, more accurately, therefore reflects the paracellular passage through the intestinal barrier with respect to the isolated measurement of urinary oligosaccharides.

The "active" results of the test depend on the size of the molecule, the mode of molecular absorption of the sugars through the paracellular route, which will therefore appear in the circulation and can be detected in the urine after renal excretion, (passive or active transporting in the intestine), the site, the rate of absorption and the distribution kinetics in the various compartments of the body. Laboratory analyses of urine samples are usually effected using high-pressure liquid chromatography (HPLC) or liquid chromatography in combination with mass spectrometry (LC/MS).

As some of the saccharides, such as lactulose, can cause increased intestinal motility, the dose administered should be kept as low as possible. The L/M permeability test is useful for assessing the permeability of the small intestine, as lactulose is degraded by bacteria in the large intestine. It is used in clinical practice, thanks to its non-invasiveness, its high sensitivity in detecting active IBD and its capacity of discriminating functional versus organic gastrointestinal disease.

<u>Outcome of the study</u>

After a daily intake of 4 capsules (cps) of lyophilized protein whey (500 mg each, 2 capsules at lunch and 2 capsules at dinner), for 8 weeks, the subjects took, at the start of the study (T0), after 30 days (T 30) and after 60 days (T 60), 50 mL of a 66.7% w/V aqueous solution of lactulose and 18% w/V of mannitol. A urine sample was consequently taken in the 5-6 hours following the intake of the L/M solution; this urine sample was analyzed in order to verify the ratio of the L/M concentrations. Observing the graphs relating to the patients' results, it was observed that the ratio between the concentration of lactulose and mannitol in the urine, both expressed in ppm, underwent a significant reduction starting from time t0, at time t1 (> 50%), up to time t2 (> 60%). This can be attributed to an effective reduction in the intra- and inter-cellular spaces following the consolidation of the tight junctions and the narrowing of the spacing between adjacent cells which make the barrier less traversable and, therefore, more selective, as can also be seen from the processing of the average values of the L/M ratio in the urines at time t0, t1 and t2, relating to the whole population recruited in the study. The standard deviation, given by the ratio between deviance (sum of the differences between the value of the L/M ratio obtained in each patient and the average value raised to the square) and degrees of freedom (total number of patients minus one), also undergoes a gradual decrease indicating a reduction in the dispersion of the data, i.e. of the L/M ratios relating to each single determination, around the average value (Figure 4).

The average values of the L/M ratio in the urines at time t0, t1 and t2, relating to a subgroup of the population that took placebo, demonstrate that the intake of the substance did not cause an effective reduction in intestinal permeability, it was observed, on the contrary, how, at time t1, an increase was actually recorded, which became re-stabilized at the values of t0, at time t2, with respect to a control group, i.e. that had taken neither the nutraceutical composition nor the placebo, see Figure 4.

<u>Conclusions</u>

The capacity was revealed of the bioactive peptides, present in the lyophilized whey waste from the production of Mozzarella di Bufala Campana PDO (lioMBCP), of inducing a significant reduction in the permeability of the small intestine, already after 30 days of treatment. The obvious sign of this result is the improvement in painful symptoms of patients suffering from IBS. The nutraceutical product based on lioMBCP is proposed as a useful support, or even a possible alternative, to classic pharmacotherapy for the treatment of patients suffering from irritable bowel syndrome.

In the future, a wide use of whey deriving from the cheese-making of MBCP by the pharmaceutical/nutraceutical industry, can be estimated. An important and unexpected upgrading of a waste product from the dairy sector is therefore expected which, to date, has limited use in the animal-feed and agro-food industry.

## Claims

1. A nutraceutical composition comprising buffalo milk whey, preferably buffalo of the Italian Mediterranean breed, wherein said composition is in the form of gastro-resistant capsules whose heads and bodies consist of 2.6 mg of titanium dioxide, 5.5 mg of gellan gum, 120.9 mg of hypromellose and are filled with 500 mg of said buffalo milk whey, wherein said buffalo milk whey is in lyophilized form.

2. The nutraceutical composition according to claims 1, wherein said buffalo milk whey is obtained from the processing of buffalo mozzarella.

3. The nutraceutical composition according to one or more of claims 1 to 2, for use in the treatment of chronic inflammatory bowel diseases, preferably in the treatment of ulcerative colitis, ulcerative rectal-colitis, Crohn's disease, irritable bowel syndrome (IBS) and/or leaky gut syndrome.

## Patentansprüche

1. Nutrazeutische Zusammensetzung, umfassend Büffelmilchmolke, vorzugsweise von Büffeln der italienischen Mittelmeerrasse, wobei die Zusammensetzung in Form von magensaftresistenten Kapseln vorliegt, deren Köpfe und Körper aus 2,6 mg Titandioxid, 5,5 mg Gellangummi, 120,9 mg Hypromellose bestehen und mit 500 mg der Büffelmilchmolke gefüllt sind, wobei die Büffelmilchmolke in lyophilisierter Form vorliegt.

2. Nutrazeutische Zusammensetzung nach Anspruch 1, wobei die Büffelmilchmolke aus der Verarbeitung von Büffelmozzarella gewonnen wird.

3. Nutrazeutische Zusammensetzung nach einem oder mehreren der Ansprüche 1 bis 2 zur Verwendung bei der Behandlung von chronisch entzündlichen Darmerkrankungen, vorzugsweise bei der Behandlung von Colitis ulcerosa, Rectal-Colitis ulcerosa, Morbus Crohn, Reizdarmsyndrom (IBS) und/oder Leaky-Gut-Syndrom.

## Revendications

1. Composition nutraceutique comprenant du lactosérum de bufflonne, de préférence de bufflonne de la race méditerranéenne italienne, dans laquelle ladite composition se présente sous la forme de capsules gastro-résistantes dont les têtes et les corps sont constitués de 2,6 mg de dioxyde de titane, 5,5 mg de gomme gellane, 120,9 mg d'hypromellose et sont remplis de 500 mg dudit lactosérum de bufflonne, dans laquelle ledit lactosérum de bufflonne se présente sous forme lyophilisée.

2. Composition nutraceutique selon la revendication 1, dans laquelle le lactosérum de bufflonne est obtenu à partir du traitement de la mozzarella de bufflonne.

3. Composition nutraceutique selon une ou plusieurs des revendications 1 à 2, pour l'utilisation dans le traitement des maladies inflammatoires chroniques de l'intestin, de préférence dans le traitement de la colite ulcéreuse, de la rectocolite ulcéreuse, de la maladie de Crohn, du syndrome du côlon irritable (IBS) et/ou du syndrome de l'intestin perméable.

Figure 1

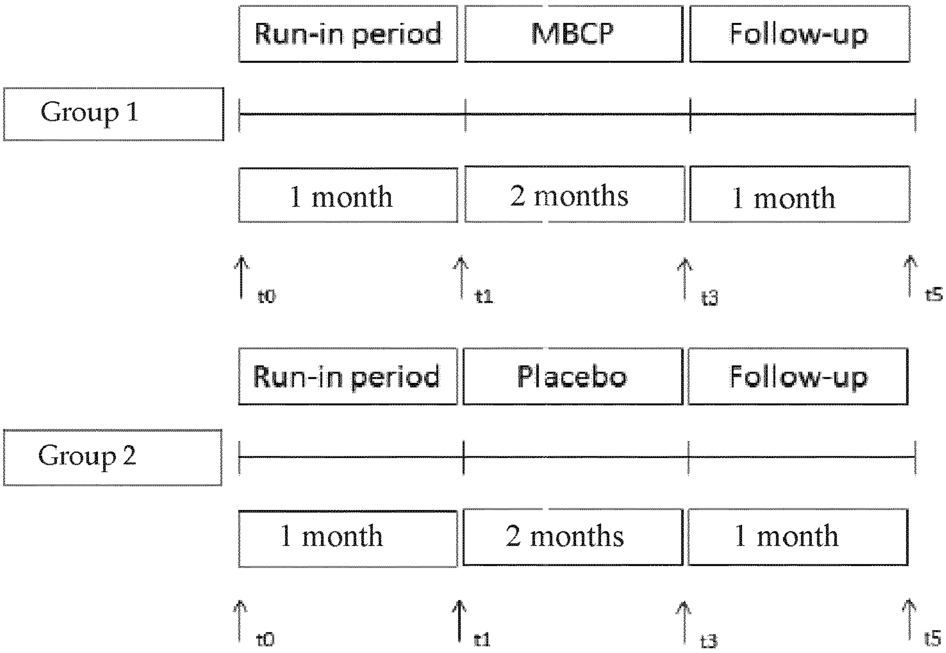


Figure 2

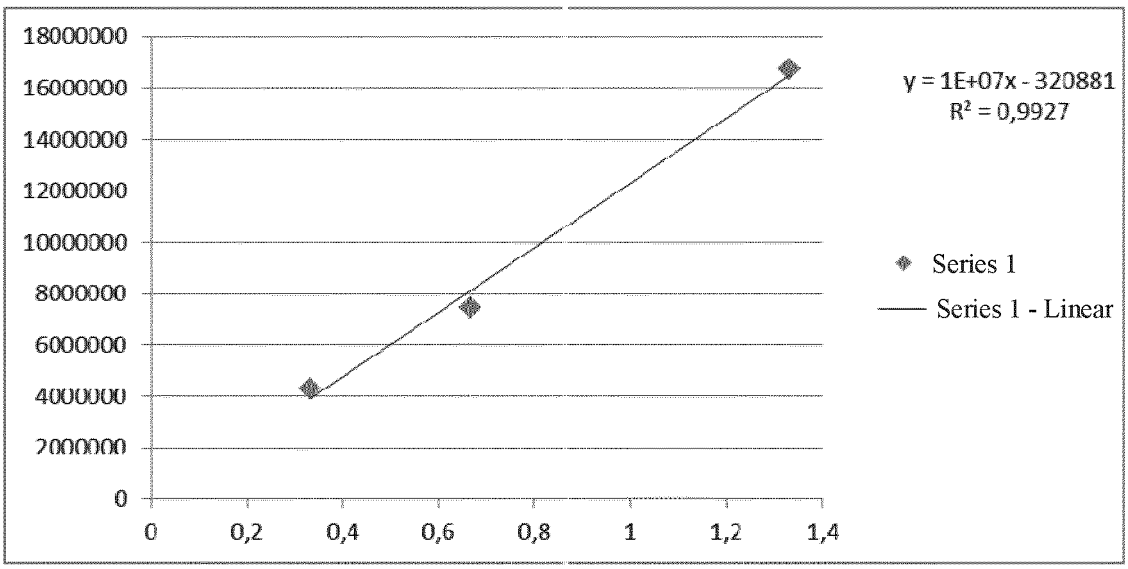

Figure 3

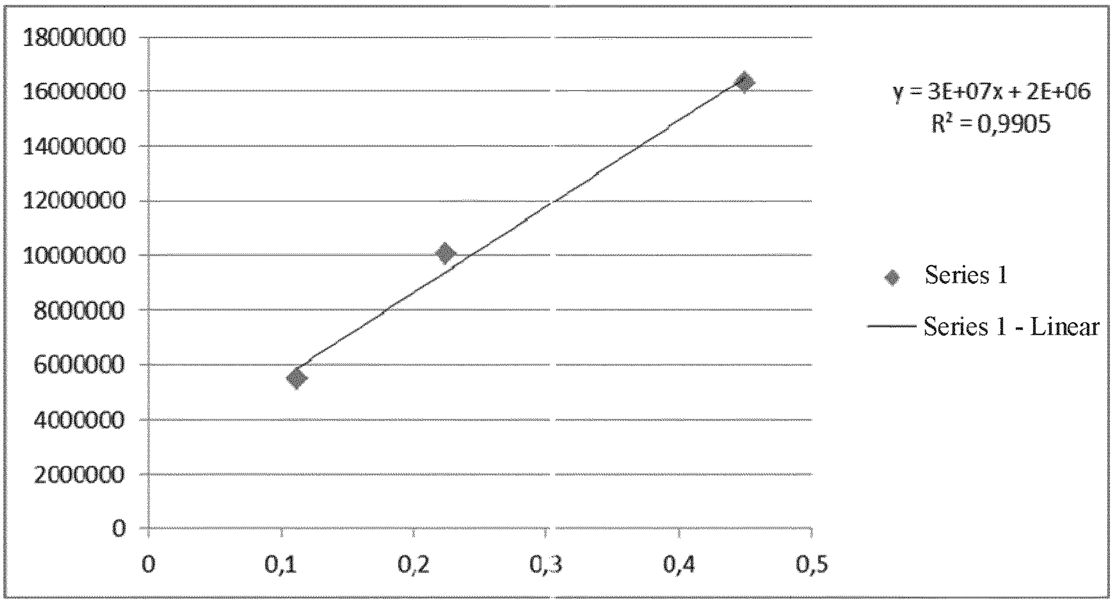


Figure 4

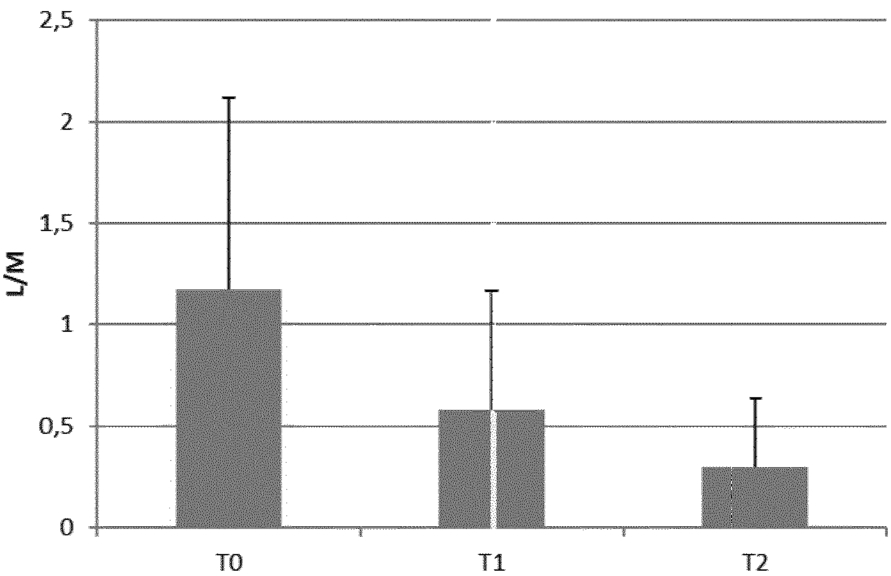


| | $t_0$ | $t_1$ | $t_2$ |
|---|---|---|---|
| Average L/M | 1,170426 | 0,581461 | 0,296511 |
| Standard deviation | 0,944734 | 0,583631 | 0,33813 |

Figure 5

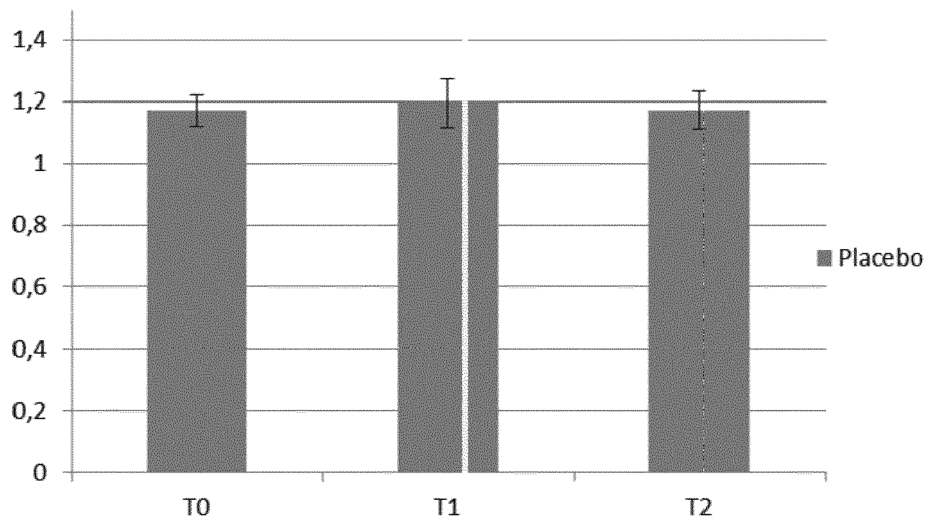

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **DE SIMONE CARMELA et al.** Characterisation and cytomodulatory properties of peptidesfrom Mozzarella di Bufala Campana cheese whey Bioactive peptides in mozzarella di Bufala whey. *Journal of peptide Science*, 26 November 2008, vol. 15 (3), ISSN 1075-2617, 251-258
- **BASILICATA MANUELA et al.** ntioxidant Properties of Buffalo-Milk Dairy Products A [beta]-Lg Peptide Released after Gastrointestinal Digestion of Buffalo Ricotta Cheese Reduces Oxidative Stress in Intestinal Epithelial Cells. *International Journal of Molecular Sciences*, 04 July 2018, vol. 19 (7), 1955
- **DE SIMONE CARMELA et al.** Peptides from water buffalo cheese whey induced senescence cell death via ceramide secretion in human colon adenocarcinoma cell line. *MOLECULAR NUTRITION & FOOD RESEARCH*, 19 August 2010, vol. 55 (2), ISSN 1613-4125, 229-238
- **TENORE GIAN et al.** Intestinal Anti-Inflammatory Effect of a Peptide Derived from Gastrointestinal Digestion of Buffalo (Bubalus buballs) Mozzarella Cheese. *Nutrients*, 13 March 2019, vol. 11 (3), 610
- Diet Capsules. DATABASE GNPD. MINTEL, 31 October 2006
- **WHITE IR** ; **CARPENTER J** ; **HORTON NJ**. Including all individuals is not enough: lessons for intention-to-treat analysis. *Clin Trials*, 2012, vol. 9, 396-407